# EUROPEAN PATENT APPLICATION

(11) **EP 3 309 246 A1**
(43) Date of publication of application: **18.04.2018**
(21) Application number: 17196546.0
(22) Date of filing: 16.10.2017
(51) Int. Cl.: C12N 1/20, C12N 9/14

(54) **MICROORGANISM INCLUDING GENETIC MODIFICATION THAT INCREASES 2-HALOACID DEHALOGENASE ACTIVITY AND METHOD OF REDUCING CONCENTRATION OF FLUORINATED METHANE IN SAMPLE BY USING THE SAME**

(30) Priority: 17.10.2016 KR 20160134549
(71) Applicant: Samsung Electronics Co., Ltd., Suwon-si, Gyeonggi-do 443-742 (KR)
(72) Inventor: Lee, Jinsuk, Suwon-si Gyeonggi-do 16678 (KR); Jung, Yukyung, Suwon-si Gyeonggi-do 16678 (KR); Park, Jinhwan, Suwon-si Gyeonggi-do 16678 (KR)
(74) Representative: Grünecker Patent- und Rechtsanwälte PartG mbB

(57) **Abstract**

Provided is a microorganism including a genetic modification that increases a 2-haloacid dehalogenase activity, a method of preparing the microorganism, and a method of using the microorganism for reducing the concentration of fluorinated methane in a sample.

## Description

### BACKGROUND

### 1. Field

The present disclosure relates to a microorganism including a genetic modification that increases a 2-haloacid dehalogenase activity, a composition for use in reducing a concentration of fluorinated methane in a sample, the composition including the microorganism, and a method of reducing the concentration of fluorinated methane in a sample.

### 2. Description of the Related Art

The emissions of greenhouse gases which have accelerated global warming are a serious environmental problem and regulations to reduce and prevent the emissions of greenhouse gases have been tightened. Among the greenhouse gases, fluorinated gases (F-gas) such as perfluorocarbons (PFCs), hydrofluorocarbons (HFCs), or sulfur hexafluoride (SF₆) show low absolute emission, but have a long half-life and a very high global warming potential, resulting in significant adverse environmental impacts. The amount of F-gas emitted from semiconductor and electronics industries, which are major causes of F-gas emission, has exceeded the assigned amount of greenhouse gas emissions and continues to increase. Therefore, costs required for degradation of greenhouse gases and greenhouse gas emission allowances are increasing every year.

A pyrolysis or catalytic thermal oxidation process has been generally used in the decomposition of F-gas. However, this process has disadvantages of limited decomposition rate, emission of secondary pollutants, high cost, etc. To solve this problem, biological decomposition of F-gas using a microbial biocatalyst has been proposed. Accordingly, there remains a need for new compositions and methods to treat F-gas in more economical and environmentally-friendly manner.

### SUMMARY

An aspect provides a microorganism including a genetic modification that increases a 2-haloacid dehalogenase (HAD) activity as compared to the same microorganism without the genetic modification, as well as a method for preparing the microorganism.

Another aspect provides a composition for use in reducing a concentration of CHₙF₄₋ₙ (where n is an integer of 0 to 3) in a sample, the composition including the microorganism including the genetic modification that increases the HAD activity.

Still another aspect provides a method of reducing a concentration of CHₙF₄₋ₙ in a sample, the method including contacting the microorganism including the genetic modification that increases the HAD activity with the sample containing CHₙF₄₋ₙ (where n is an integer of 0 to 3) to reduce the concentration of CHₙF₄₋ₙ (where n is an integer of 0 to 3) in the sample.

### BRIEF DESCRIPTION OF THE DRAWINGS

These and/or other aspects will become apparent and more readily appreciated from the following description of the embodiments, taken in conjunction with the accompanying drawings in which:
FIG. 1 shows a vector map of a pET-BC HAD vector; and
FIG. 2 shows a vector map of a pET-BG HAD vector.

### DETAILED DESCRIPTION

The terms "increase in activity" or "increased activity" or "increases activity" and the like, as used herein, may refer to a detectable increase in an activity of a cell, a protein, or an enzyme. The "increase in activity" or "increased activity" may also refer to an activity level of a modified (e.g., genetically engineered) cell, protein, or enzyme that is higher than that of a comparative cell, protein, or enzyme of the same type, such as a cell, protein, or enzyme that does not have a given genetic modification (e.g., original or "wild-type" cell, protein, or enzyme). The phrase "activity of a cell" may refer to an activity of a particular protein or enzyme of a cell. For example, an activity of a modified or engineered cell, protein, or enzyme may be increased by about 5% or more, about 10% or more, about 15% or more, about 20% or more, about 30% or more, about 50% or more, about 60% or more, about 70% or more, or about 100% or more relative to an activity of a non-engineered cell, protein, or enzyme of the same type, i.e., a wild-type cell, protein, or enzyme. A cell having an increased activity of a protein or an enzyme may be identified by using any method known in the art.

An increase in an activity of an enzyme or a polypeptide may be achieved by an increase in expression or specific activity. The increase in expression may be caused by introduction of a polynucleotide encoding the enzyme or the polypeptide into a cell; by otherwise increasing of the copy number of a gene encoding the enzyme or polypeptide, by modification of a regulatory region of the polynucleotide encoding the enzyme or polypeptide. A microorganism into which the gene is introduced may be a microorganism endogenously including the gene (e.g., the exogenous gene is homologous to the microorganism), or a microorganism that does not endogenously include the gene (e.g., the exogenous gene is heterologous to the microorganism). The gene may be operably linked to a regulatory sequence that allows expression thereof, for example, a promoter, an enhancer, a polyadenylation site, or a combination thereof. The polynucleotide whose copy number is increased may be endogenous or exogenous. An endogenous gene is a gene that exists in the genetic material of a microorganism prior to the genetic modification that increases the activity of the enzyme or polypeptide. An exogenous gene refers to a gene that is introduced into a cell in the genetic modification, and may be homologous or heterologous with respect to a host cell into which the gene is introduced. The term "heterologous" means "foreign" or "not native." Thus, an "exogenous" gene can be introduced despite the preexistence of the same or similar gene in the microorganism.

An "increase of the copy number" may be caused by introduction of an exogenous gene or amplification of an endogenous gene. Optionally, the increase in copy number achieved by genetically engineering a cell to introduce a gene that does not exist in a non-engineered cell. The introduction of the gene may be mediated by a vehicle such as a vector. The introduction may be via a transient introduction in which the gene is not integrated into a genome (e.g., in an episome such as a plasmid), or via integration of the gene into the genome. The introduction may be performed, for example, by introducing a vector into the cell, the vector including a polynucleotide encoding a target polypeptide, and then, replicating the vector in the cell, or by integrating the polynucleotide into the genome.

The introduction of the gene may be performed via a known method, for example, transformation, transfection, or electroporation. The gene may be introduced via a vehicle or as it is. The term "vehicle", as used herein, refers to a nucleic acid molecule that is able to deliver other nucleic acids linked thereto. As a nucleic acid sequence mediating introduction of a specific gene, the vehicle used herein is construed to be interchangeable with a vector, a nucleic acid construct, and a cassette. The vector may include, for example, a plasmid vector, a virus-derived vector, etc. The plasmid can be a circular doublestranded DNA molecule linkable with another DNA. The vector may include, for example, a plasmid expression vector, a virus expression vector, such as a replication-defective retrovirus, adenovirus, adeno-associated virus, or a combination thereof.

The genetic modification used in the present disclosure may be performed by a molecular biological method known in the art.

The term "parent cell" refers to an original cell or a cell of the same type as an original cell from which a genetically engineered cell is produced. With respect to a particular genetic modification, the "parent cell" may be a cell (whether previously engineered or not) that lacks the particular genetic modification, but is identical in all other respects. Thus, the parent cell may be a cell that is used as a starting material to produce a genetically engineered microorganism having increased activity of a given protein (e.g., a protein having a sequence identity of about 95% or higher amino acid identity with respect to 2-haloacid dehalogenase). The same comparison is also applied to other genetic modifications. In some embodiments, the parent cell can be a wild-type cell.

The term "gene", as used herein, refers to a nucleotide fragment expressing a particular protein, and may or may not be operably linked to a regulatory sequence (e.g., a 5'-non coding sequence and/or a 3'-non coding sequence).

The term "sequence identity" of a polynucleotide or a polypeptide, as used herein, refers to a degree of identity between bases or amino acid residues of sequences obtained after the sequences are aligned so as to best match in certain comparable regions. The sequence identity is a value that is measured by comparing two sequences in certain comparable regions via optimal alignment of the two sequences, in which portions of the sequences in the certain comparable regions may be added or deleted compared to reference sequences. A percentage of sequence identity may be calculated by, for example, comparing two optimally aligned sequences in the entire comparable regions, determining the number of locations in which the same amino acids or nucleic acids appear to obtain the number of matching locations, dividing the number of matching locations by the total number of locations in the comparable regions (that is, the size of a range), and multiplying a result of the division by 100 to obtain the percentage of the sequence identity. The percentage of the sequence identity may be determined using a known sequence comparison program, for example, BLASTN (NCBI), BLASTP (NCBI), CLC Main Workbench (CLC bio), MegAlign™ (DNASTAR Inc),), or EMBOSS Needle. For example, the Needleman-Wunsch global alignment algorithm in EMBOSS may be used with the default settings (gap opening penalty 10, gap extension penalty 0.5; end gap penalty =false, end gap open-10, for amino acid sequence comparisons, the BLOSUM62 matrix is used).

Various levels of sequence identity may be used to identify various types of polypeptides or polynucleotides having the same or similar functions or activities. For example, the sequence identity may include a sequence identity of about 50% or more, about 55% or more, about 60% or more, about 65% or more, about 70% or more, about 75% or more, about 80% or more, about 85% or more, about 90% or more, about 95% or more, about 96% or more, about 97% or more, about 98% or more, about 99% or more, or 100%.

Where a polynucleotide sequence encoding a given protein, other polynucleotide sequences can be substituted based on the degeneracy of the genetic code.

The term "genetic modification", as used herein, refers to an artificial alteration in a constitution or structure of a genetic material of a cell.

In the present disclosure, % represents w/w%, unless otherwise mentioned.

An aspect of the disclosure provides a microorganism including a genetic modification that increases a 2-haloacid dehalogenase (HAD) activity.

2-Haloacid dehalogenase is an enzyme that catalyzes a chemical reaction of 2-haloacid+H₂O ⇔ 2-hydroxyacid+halide. Thus, two substrates of this enzyme are 2-haloacid and H₂O, and its two products are 2-hydroxyacid and halide. This enzyme belongs to the family of hydrolases which act on halide bonds in carbon-halide compounds. However, with regard to reducing a concentration of CHₙF₄₋ₙ (where n is an integer of 0 to 3) in a sample, the microorganism should not be construed as being limited to this particular mechanism. The HAD may belong to EC 3.8.1.2. The HAD may be exogenous or endogenous. The HAD may be selected from the group consisting of HADs derived from the genus *Bacillus, Pseudomonas, Azotobacter, Agrobacterium,* and *Escherichia.* The HAD may be derived from a strain selected from the group consisting of *Bacillus cereus, Bacillus thuringiensis,* and *Bacillus megaterium.*

The HAD may be a polypeptide having a sequence identity of about 95% or more with respect to amino acid sequences of SEQ ID NOS: 1, 3, 5, 7, 9, and 11.

With regard to the above microorganism, the genetic modification may increase expression of a gene encoding the HAD. The genetic modification may increase the copy number of the HAD gene. The genetic modification may increase the copy number of one or more genes among genes encoding polypeptides having a sequence identity of 95% or more with respect to the amino acid sequences of SEQ ID NOS: 1, 3, 5, 7, 9, and 11, respectively. The genes may have a sequence identity of 95% or more with respect to the nucleotide sequences of SEQ ID NOS: 2, 4, 6, 8, 10, and 12, respectively. The genetic modification may be the introduction an exogenous gene encoding HAD, for example, via a vehicle such as a vector. The gene encoding HAD may exist within or outside the chromosome. The introduced gene encoding HAD may be a plurality of genes (e.g., replicates of the same gene and/or a mixture of genes encoding multiple gene variants), for example, 2 or more, 5 or more, 10 or more, 50 or more, 100 or more, or 1000 or more.

The microorganism may belong to the genus *Escherichia, Bacillus, Xanthobacter, Pseudomonas, Azotobacter,* or *Agrobacterium.* The microorganism may be, for example, of the species *E. coli* or *X. autotrophicus.*

The microorganism may further include one or more genes selected from the group consisting of a gene encoding ArgU and a gene encoding ProL. The gene encoding ArgU may encode tRNA that recognizes the arginine codons, AGA and AGG. The gene encoding ProL may encode tRNA that recognizes the proline codon, CCC. The microorganism may be BL21-CodonPlus(DE3)-RP (or another strain having similar codon bias reducing features) which further includes both of the gene encoding ArgU and the gene encoding ProL.

The microorganism may further include, or be modified to include, one or more genes selected from the group consisting of the gene encoding ArgU, a gene encoding ileY, and a gene encoding ileW. The gene encoding ileY may encode tRNA that recognizes the isoleucine codon, AUA. The gene encoding ileW may encode tRNA that recognizes the leucine codon, CUA. The microorganism may be BL21-CodonPlus-RIL or BL21-CodonPlus(DE3)-RIL which further includes all of the genes encoding ArgU, the gene encoding ileY, and the gene encoding ileW.

The microorganism may reduce a concentration of CHₙF₄₋ₙ (where n is an integer of 0 to 3) (referred to herein as "fluorinated methane") in a sample. The reducing may be performed by introducing a hydroxyl group to carbon of the fluorinated methane by action of the HAD enzyme protein on C-F or C-H bond thereof or by accumulating the fluorinated methane inside the cell of the microorganism. Further, the reducing may include cleaving of C-F bonds of CHₙF₄₋ₙ, converting of CHₙF₄₋ₙ into other materials, or intracellular accumulating of CHₙF₄₋ₙ. The sample may be in a liquid or gas state. The sample may be industrial waste water or waste gas. The sample may be any sample including the fluorinated methane. The fluorinated methane may include CF₄, CHF₃, CH₂F₂, CH₃F, or a mixture thereof.

Reducing CHₙF₄₋ₙ in the sample encompasses a reduction in CHₙF₄₋ₙ to any degree, including complete removal of CHₙF₄₋ₙ. The sample may be a gas or a liquid. The composition may further include a material that increases solubility of the fluorinated methane for a medium or a culture.

Another aspect provides a composition used for reducing a concentration of CHₙF₄₋ₙ (where n is an integer of 0 to 3) in a sample, the composition including the microorganism including the genetic modification that increases 2-haloacid dehalogenase (HAD) activity. Still another aspect provides a method of reducing a concentration of CHₙF₄₋ₙ in a sample, the method including contacting the microorganism with the sample containing CHₙF₄₋ₙ (where n is an integer of 0 to 3) to reduce the concentration of CHₙF₄₋ₙ (where n is an integer of 0 to 3) in the sample, in which the microorganism includes the genetic modification that increases 2-haloacid dehalogenase (HAD) activity. The microorganism, sample and fluorinated methane are the same as described above.

The composition or method may reduce the concentration of fluorinated methane in the sample by contacting the composition with the sample. The contacting may be performed in a liquid or solid phase. The contacting may be performed, for example, by contacting a culture of the cultured microorganism with the sample during culturing. Optionally, the culturing may be performed under conditions where the microorganism may proliferate. In some embodiments, the contacting may be performed in a sealed container (e.g., one that prevents the escape of fluorinated gas). The phrase "sealed container" represents an air tight condition. The contacting may be performed in a closed, flow-through type system, permitting continuous flow of gas. The contacting may include culturing or incubating the microorganism while contacting it with a sample containing CHₙF₄₋ₙ (where n is an integer of 0 to 3). The contacting may include culturing the microorganism under conditions where the microorganism may survive or proliferate, or conditions where the microorganism may be allowed to be in a resting state. The contacting may be performed when the growth stage of the microorganism is in an exponential phase or a stationary phase. The culturing may be performed under aerobic or anaerobic conditions.

The sample may be in a liquid or gas state. The sample may be industrial waste water or waste gas. The sample may include passive contacting of the sample with the culture of the microorganism and active contacting of the sample with the culture of the microorganism. The sample may be, for example, sparged into the culture of the microorganism. That is, the sample may be sparged into a medium or culture. The sparging may be sparging of the sample from the bottom to the top of the medium or culture. The sparging may be via injecting of droplets or bubbles of the sample.

The contacting of the microorganism with the sample may be performed in a batch or continuous manner. The contacting may include, for example, contacting a fresh microorganism including the genetic modification that increases HAD activity with a sample obtained in a previous contacting step. In other words, the method can comprise repeatedly contacting the sample with microorganisms, such that the sample is contacted with fresh microorganisms twice or more, for example, twice, three times, five times, or ten times or more. Alternately or in addition, fresh microorganism may be contacted with the sample by continuous infusion of fresh microorganism and, optionally, continuous removal of used microorganism. The term "fresh" microorganism means a microorganism that has not yet been contacted with the sample. "Used" microorganism means a microorganism that has been contacted with the sample. The contacting may be continued or repeated until the concentration of fluorinated methane in the sample reaches a desired reduced concentration.

Still another aspect provides a method of producing the microorganism, the method including introducing into the microorganism the genetic modification that increases 2-haloacid dehalogenase (HAD) activity. The method may be a method of producing the microorganism, including introducing the gene encoding HAD into the microorganism. The introducing of the gene encoding HAD may be achieved by introducing of a vehicle including an exogenous gene into the microorganism. With regard to the method, the genetic modification may include amplification of the gene, manipulation of the regulatory sequence of the gene, or manipulation of the sequence of the gene itself. The manipulation may be insertion, substitution, conversion, or addition of nucleotides.

The method may further include increasing the copy number of one or more genes selected from the group consisting of the gene encoding ArgU and the gene encoding ProL in the microorganism. The method may further include increasing the copy number of one or more genes selected from the group consisting of the gene encoding ArgU, the gene encoding ileY, and the gene encoding ileW in the microorganism.

The microorganism according to an aspect may be used to remove CHₙF₄₋ₙ in the sample.

The composition according to another aspect may be used to remove CHₙF₄₋ₙ in the sample.

The method of reducing the concentration of CHₙF₄₋ₙ in the sample according to still another aspect may be used to efficiently reduce the concentration of CHₙF₄₋ₙ in the sample.

Reference will now be made in detail to embodiments, examples of which are illustrated in the accompanying drawings, wherein like reference numerals refer to like elements throughout. In this regard, the present embodiments may have different forms and should not be construed as being limited to the descriptions set forth herein. Accordingly, the embodiments are merely described below, by referring to the figures, to explain aspects. As used herein, the term "and/or" includes any and all combinations of one or more of the associated listed items. These Examples are provided for illustrative purposes only, and the invention is not intended to be limited by these Examples.

### Example 1: Decomposition of CF₄ by Bacillus Strain

*Bacillus cereus* (KCTC 3624) and *Bacillus megaterium* (DSM 32) strains were cultured in LB medium under shaking at 30°C and 230 rpm until cell density reached OD₆₀₀ = 3.0. 10 ml of each cell culture was added to a 60 ml-serum bottle, and the bottles were sealed. The LB medium included 10 g of tryptone, 5 g of yeast extract, and 10 g of NaCl per 1 L of distilled water.

Next, gas-phase CF₄ was injected through a rubber stopper of a cap of the serum bottle using a syringe to a headspace concentration of 1000 ppm. Thereafter, the serum bottles were incubated for 5 days under shaking at 30°C and 230 rpm. Each experiment was performed in triplicate.

After incubation, 0.5 ml of the headspace gas containing no medium in the serum bottle was collected using a 1.0 ml-headspace syringe and injected into GC (Agilent 7890, Palo Alto, CA, USA). The injected headspace sample was separated through a CP-PoraBOND Q column (25m length, 0.32mm i.d., 5 um film thickness, Agilent), and changes in the CF₄ concentration were analyzed by mass spectrometry (Agilent 5973, Palo Alto, CA, USA). As a carrier gas, helium was used, and applied to the column at a flow rate of 1.5 ml/min. Gas chromatography (GC) conditions were as follows: The inlet temperature was 250°C, initial temperature was maintained at 40°C for 2 minutes, then raised to 290°C at a rate of 20°C/min. Mass spectrometry conditions were as follows: Ionization energy was 70 eV, interface temperature was 280°C, ion source temperature was 230°C, and quadrupole temperature was 150°C.

Table 1 shows percentages of residual CF₄ in the samples when *B. cereus* and *B. megaterium* strains were cultured under the conditions as above. As shown in Table 1, *B. cereus* and *B. megaterium* strain cultures showed about 6.63% and about 9.33% decreases in the headspace concentrations of CF₄, compared to a control group containing only LB medium, respectively.

**[Table 1]**

| Strain | Residual CF₄(%) |
|---|---|
| Control (medium) | 100.00 |
| *B. cereus* | 93.37 |
| *B. megaterium* | 90.67 |

### Example 2: Preparation of Recombinant E. coli introduced with Bacillus cereus-derived HAD gene and Decomposition of CF₄ by Using the Same

### 1. Amplification of Bacillus cereus-derived HAD gene (BC HAD) and Introduction of the gene into E. coli

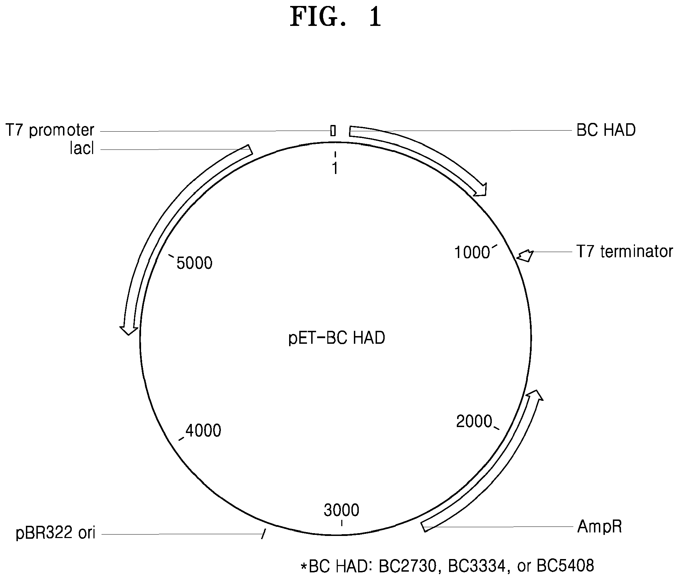

*B. cereus* (KCTC 3624) was cultured in an LB medium at 30°C under shaking at 230 rpm overnight, and then genomic DNA was isolated using the total DNA extraction kit (Invitrogen Biotechnology). PCR was performed using this genomic DNA as a template and a set of primers having nucleotide sequences given in Table 2 to amplify and obtain BC2730, BC3334, and BC5408 genes, respectively. The BC HAD genes thus amplified were ligated with pETDuet-1 (Novagen, Cat. No. 71146-3), respectively which was digested with restriction enzymes, Ncol and HindIII, using the InFusion Cloning Kit (Clontech Laboratories, Inc.) to prepare 3 kinds of pET-BC HAD vectors. FIG. 1 shows a vector map of the pET-BC HAD vector. BC2730, BC3334, and BC5408 have amino acid sequences of SEQ ID NOS: 1, 3, and 5, respectively. Their genes have nucleotide sequences of SEQ ID NOS: 2, 4, and 6, respectively.

Next, each of the prepared three kinds of pET-BC HAD vectors, pET-BC2730, pET-BC3334, and pET-BC5408 was introduced to *E. coli* BL21 by a heat shock method, and then cultured on a LB plate containing 100 µg/mL ampicillin. Strains showing ampicillin resistance were selected. Finally, three strains thus selected were designated as a recombinant *E. coli* BL21/pET-BC2730, BL21/pET-BC3334, and BL21/pET-BC5408, respectively.

**[Table 2]**

| BC HAD gene | Primer sequence (SEQ ID NO) |
|---|---|
| BC2730 | Forward: SEQ ID NO: 13 |
| | Reverse: SEQ ID NO: 14 |
| BC3334 | Forward: SEQ ID NO: 15 |
| | Reverse: SEQ ID NO: 16 |
| BC5408 | Forward: SEQ ID NO: 17 |
| | Reverse: SEQ ID NO: 18 |

### 2. Decomposition of perfluoromethane by BC HAD gene-introduced E. coli

In this section, it was examined whether the three kinds of recombinant *E. coli* BL21/pET-BC HAD strains prepared in section (1) affect removal of CF₄ in a sample.

In detail, each of BL21/pET-BC2730, BL21/pET-BC3334, and BL21/pET-BC5408 was cultured in a TB medium at 30°C under shaking at 230 rpm. At OD₆₀₀ of about 0.5, 0.2 mM of IPTG was added thereto, followed by culturing at 20°C under shaking 230 rpm overnight. Each of the cells was harvested and suspended in an LB medium to a cell density of OD₆₀₀ of 3.0. 10 ml of each cell suspension was added to a 60 ml-serum bottle, and then the bottles were sealed. The LB medium has the same composition as in Example 1.

Next, gas-phase CF₄ was injected through a rubber stopper of a cap of the serum bottle using a syringe to its headspace concentration of 1000 ppm. Thereafter, the serum bottle was incubated for 4 days under shaking at 30°C and 230 rpm. Each experiment was performed in triplicate. After incubation, a headspace concentration of CF₄ in the serum bottle was analyzed under the same conditions as in Example 1.

Table 3 shows percentages of residual CF₄ in the samples when the recombinant *E. coli* BL21/pET-BC HAD strains were cultured under the conditions as above. As shown in Table 3, the recombinant E. coli strains introduced with BC HAD (BC2730, BC3334, or BC5408) showed about 1.69% decrease, about 5.67% decrease, and about 5.44% decrease in the headspace concentrations of CF₄, compared to a control group introduced with an empty vector.

**[Table 3]**

| Recombinant strain | Residual CF₄(%) |
|---|---|
| Control (empty vector) | 100.00 |
| BC2730 | 98.31 |
| BC3334 | 94.33 |
| BC5408 | 94.56 |

### Example 3: Preparation of Recombinant E. coli introduced with Bacillus megaterium-derived HAD gene and Decomposition of CF₄ by Using the Same

### 1. Amplification of Bacillus megaterium-derived HAD gene (BG HAD) and Introduction of the gene into E. coli

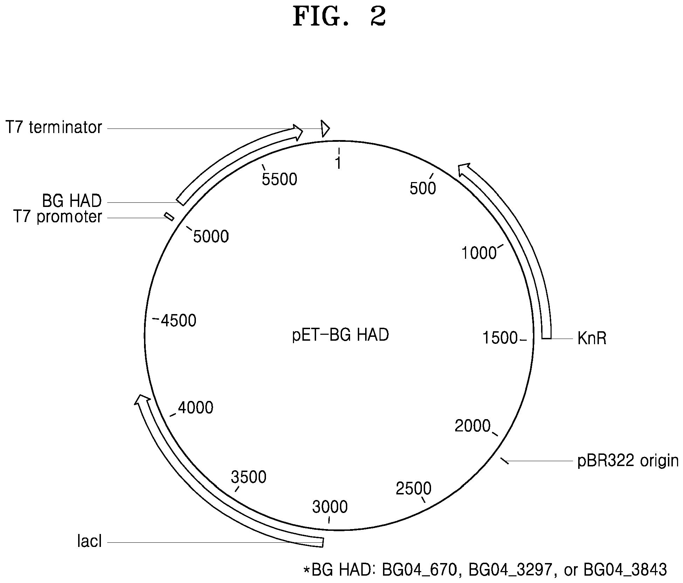

*B. megaterium* (DSM 32) was cultured in LB medium at 30°C under shaking at 230 rpm overnight, and then genomic DNA was isolated using the total DNA extraction kit (Invitrogen Biotechnology). PCR was performed using this genomic DNA as a template and a set of primers having nucleotide sequences given in Table 4 to amplify and obtain BG04_670, BG04_3297, and BG04_3843 genes, respectively. BG04_670, BG04_3297, and BG04_3843 have amino acid sequences of SEQ ID NOS: 7, 9, and 11, respectively. Their genes have nucleotide sequences of SEQ ID NOS: 8, 10, and 12, respectively. The BG HAD genes thus amplified were ligated with pET28a (Novagen, Cat. No. 69864-3), respectively which was digested with restriction enzymes, Ncol and Xhol, using the InFusion Cloning Kit (Clontech Laboratories, Inc.) to prepare 3 kinds of pET-BG HAD vectors. FIG. 2 shows a vector map of the pET-BG HAD vector.

Next, each of the prepared three kinds of pET-BG HAD vectors, pET-BG04_670, pET-BG04_3297, pET-BG04_3843 was introduced to *E. coli* BL21 by a heat shock method, and then cultured on a LB plate containing 50 µg/mL kanamycin. Strains showing kanamycin resistance were selected. Finally, three strains thus selected were designated as a recombinant *E. coli* BL21/pET-BG04_670, BL21/pET-BG04_3297, and BL21/pET-BG04_3843, respectively.

**[Table 4]**

| BG HAD gene | Primer sequence (SEQ ID NO) |
|---|---|
| BG04_670 | Forward: SEQ ID NO: 19 |
| | Reverse: SEQ ID NO: 20 |
| BG04_3297 | Forward: SEQ ID NO: 21 |
| | Reverse: SEQ ID NO: 22 |
| BG04_3843 | Forward: SEQ ID NO: 23 |
| | Reverse: SEQ ID NO: 24 |

### Decomposition of perfluoromethane by BG HAD gene-introduced E. coli

In this section, it was examined whether the three kinds of recombinant *E. coli* BL21/pET-BG HAD strains prepared in section (1) effect removal of CF₄ in a sample.

In detail, each of *E. coli* BL21/pET-BG04_670, BL21/pET-BG04_3297, and BL21/pET-BG04_5408 strains was cultured in a TB medium at 30°C under shaking at 230 rpm. At OD₆₀₀ of about 0.5, 0.2 mM of IPTG was added thereto, followed by culturing at 20°C under stirring 230 rpm overnight. Each of the cells was harvested and suspended in LB medium to a cell density of OD₆₀₀ of 3.0. 10 ml of each cell suspension was added to a 60 ml-serum bottle, and then the bottles were sealed. The LB medium has the same composition as in Example 1.

Next, gas-phase CF₄ was injected through a rubber stopper of a cap of the serum bottle using a syringe to its headspace concentration of 1000 ppm. Thereafter, the serum bottle was incubated for 4 days under shaking at 30°C and 230 rpm. Each experiment was performed in triplicate. After incubation, a headspace concentration of CF₄ in the serum bottle was analyzed under the same conditions as in Example 1.

Table 5 shows percentages of residual CF₄ in the samples when the recombinant *E. coli* BL21/pET-BG HAD strains were cultured under the conditions as above. As shown in Table 5, the recombinant *E. coli* strains introduced with BG HAD (BG04_670, BG04_3297, or BG04_3843) showed about 6.01% decrease, about 8.55% decrease, and about 8.95% decrease in the headspace concentrations of CF₄, compared to a control group introduced with an empty vector.

**[Table 5]**

| Recombinant strain | Residual CF₄(%) |
|---|---|
| Control (empty vector) | 100.00 |
| BG04_670 | 93.99 |
| BG04_3297 | 91.45 |
| BG04_3843 | 91.05 |

The use of the terms "a" and "an" and "the" and "at least one" and similar referents in the context of describing the invention (especially in the context of the following claims) are to be construed to cover both the singular and the plural, unless otherwise indicated herein or clearly contradicted by context. The use of the term "at least one" followed by a list of one or more items (for example, "at least one of A and B") is to be construed to mean one item selected from the listed items (A or B) or any combination of two or more of the listed items (A and B), unless otherwise indicated herein or clearly contradicted by context. The terms "comprising," "having," "including," and "containing" are to be construed as open-ended terms (i.e., meaning "including, but not limited to,") unless otherwise noted. Recitation of ranges of values herein are merely intended to serve as a shorthand method of referring individually to each separate value falling within the range, unless otherwise indicated herein, and each separate value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein, is intended merely to better illuminate the invention and does not pose a limitation on the scope of the invention unless otherwise claimed. No language in the specification should be construed as indicating any non-claimed element as essential to the practice of the invention.

Preferred embodiments of this invention are described herein, including the best mode known to the inventors for carrying out the invention. Variations of those preferred embodiments may become apparent to those of ordinary skill in the art upon reading the foregoing description. The inventors expect skilled artisans to employ such variations as appropriate, and the inventors intend for the invention to be practiced otherwise than as specifically described herein.

## Claims

1. A microorganism comprising a genetic modification that increases a 2-haloacid dehalogenase (HAD) activity as compared to a microorganism of the same type without the genetic modification.

2. The microorganism of Claim 1, wherein the genetic modification increases the copy number of a gene encoding the HAD, or the genetic modification is the introduction of an exogenous gene encoding HAD.

3. The microorganism of Claim 1 or 2, wherein the HAD is from *Bacillus, Pseudomonas, Azotobacter, Agrobacterium,* or *Escherichia*; preferably wherein the HAD is from *Bacillus cereus, Bacillus thuringiensis,* or *Bacillus megaterium.*

4. The microorganism of any one of Claims 1 to 3, wherein the HAD belongs to EC 3.8.1.2.

5. The microorganism of any one of Claims 1 to 4, wherein the HAD is a polypeptide having a sequence identity of 95% or more with respect to an amino acid sequence of SEQ ID NO: 1, 2, 3, 4, 5, or 6.

6. The microorganism of any one of Claims 2 to 5, wherein the gene has a sequence identity of 95% or more with respect to a nucleotide sequence of SEQ ID NO: SEQ ID NO: 7, 8, 9, 10, 11, or 12.

7. The microorganism of any one of Claims 1 to 6, wherein the microorganism belongs to the genus *Escherichia, Bacillus,* or *Xanthobacter.*

8. The microorganism of any one of Claims 1 to 6, wherein the genetic modification is the introduction of an exogenous gene encoding HAD from *Bacillus,* and the microorganism belongs to the genus of *Escherichia.*

9. A composition used for reducing a concentration of CHₙF₄₋ₙ (where n is an integer of 0 to 3) in a sample, comprising a microorganism of any one of claims 1 to 8.

10. The composition of Claim 9, wherein the reducing comprises cleaving of C-F bonds of CHₙF₄₋ₙ (where n is an integer of 0 to 3), converting of CHₙF₄₋ₙ into other materials, or intracellular accumulating of CHₙF₄₋ₙ.

11. A method of reducing a concentration of CHₙF₄₋ₙ in a sample, the method comprising contacting the microorganism of any one of claims 1 to 8 with a sample containing CHₙF₄₋ₙ (where n is an integer of 0 to 3) to reduce the concentration of CHₙF₄₋ₙ (where n is an integer of 0 to 3) in the sample.

12. The method of Claim 11, wherein the microorganism is contacted with the sample in a sealed container.

13. The method of Claim 11 or 12, wherein contacting of the microorganism with the sample comprises culturing or incubating the microorganism while in contact with the sample containing CHₙF₄₋ₙ (where n is an integer of 0 to 3).

14. The method of Claim 12, wherein the method comprises culturing the microorganism in the sealed container under conditions in which the microorganism proliferates.

15. A method of preparing a microorganism of any one of claims 1 to 8, which method comprises introducing into a microorganism an exogenous gene encoding an HAD.


<110>    Samsung Electronics Co., Ltd.

<120>    Microorganism including genetic modification that increases
         activity of 2-haloacid dehalogenase and method for reducing
         concentration of fluorinated methane in sample

<130>    PN114712KR

<160>    24

<170>    KopatentIn 2.0

<210>    1
<211>    231
<212>    PRT
<213>    Bacillus cereus

<400>    1
Met Met Gly Tyr Lys Ala Met Leu Phe Asp Leu Asp Asp Thr Leu Leu
  1               5                  10                  15

Asp Arg Asp Lys Ala Val Glu Ala Leu Phe Leu Ile Val Leu Glu Lys
             20                  25                  30

Cys Tyr Glu Asn Val Asp Gly Ala Ala Lys Ser Asn Met Leu Gln Lys
         35                  40                  45

Phe Lys Glu Tyr Asp Lys Arg Glu Tyr Gly Ile Ser Asn Lys Thr Thr
     50                  55                  60

Val Leu Glu Ser Leu Phe Asp Glu Phe Thr Pro Arg Tyr Arg Leu Pro
 65                  70                  75                  80

Arg Asn Tyr Ile Gln Asp Phe Trp Asn Asn Asn Phe Pro Arg Cys Phe
                 85                  90                  95

Ser Ile Asp Gln Asn Thr Ile His Phe Leu Asn Gln Ile Lys Lys His
            100                 105                 110

Cys Lys Val Gly Ile Ile Thr Asn Gly Ser Thr Gln Arg Gln Lys Ala
        115                 120                 125

Lys Ile Phe Asn Thr Asn Leu Asn Lys Tyr Phe Glu Thr Ile Ile Ile
    130                 135                 140

Ser Glu Glu Val Gly Phe Ser Lys Pro Asp Lys Arg Ile Phe Glu Leu
145                 150                 155                 160

Ala Leu Asn Lys Leu Asn Leu Gln Pro Glu Asn Thr Leu Phe Val Gly
                165                 170                 175

Asp Asp Leu Glu Lys Asp Ile Ala Gly Pro Gln Asn Ala Asn Ile Lys
            180                 185                 190

Gly Val Trp Phe Asn Pro Gln Lys Ile Lys Asn Thr Thr Lys Ile Gln
        195                 200                 205

Pro Tyr Ala Glu Ile Asn Thr Leu Asp Ser Leu Leu Ser Tyr Val Thr
    210                 215                 220

Pro Gln Tyr Phe Tyr Asn Lys
225                 230

<210>    2
<211>    696
<212>    DNA
<213>    Bacillus cereus

<400>    2
atgatgggtt ataaagcgat gctgtttgat ttagatgata cattacttga tagggataaa        60

gcagtagagg cattattttt aattgtttta gaaaagtgtt atgaaaatgt agatggtgcg       120

gctaaaagca acatgttaca gaaattcaaa gaatacgata aaagagaata tggtataagt       180

aataaaacga cagttttaga atcattgttt gatgaattca cgccaaggta tagattgcca       240

cgcaattaca tccaagattt ttggaataat aatttcccta gatgtttttc aatagaccaa       300

aatactattc atttcttaaa tcaaataaag aagcactgta aagttggaat tataacaaat       360

ggctcaactc agaggcaaaa agctaaaata tttaacacga atttaaataa gtattttgaa       420

acaatcatta tttctgaaga agtgggattt agtaaacctg ataaacgtat attcgaacta       480

gcattaaata agttaaattt acaaccggaa aatactttat tcgttgggga tgacttagaa       540

aaggatattg ctggtcctca aaatgcaaat ataaaaggtg tgtggtttaa ccctcagaaa       600

atcaagaata ctaccaaaat acaaccatat gccgagatta acactttgga tagtttgtta       660

agttatgtta ctccacaata tttttataac aagtaa                                 696


<210>    3
<211>    236
<212>    PRT
<213>    Bacillus cereus

<400>    3
Met Lys Tyr Lys Val Ile Leu Phe Asp Val Asp Asp Thr Leu Leu Asp
1               5                   10                  15

Phe Pro Glu Thr Glu Arg His Ala Leu His Asn Ala Phe Val Gln Phe
            20                  25                  30

Asp Met Pro Thr Gly Tyr Asn Asp Tyr Leu Ala Ser Tyr Lys Glu Ile
        35                  40                  45

Ser Asn Gly Leu Trp Arg Asp Leu Glu Asn Lys Met Ile Thr Leu Ser
    50                  55                  60

Glu Leu Ala Val Asp Arg Phe Arg Gln Leu Phe Ala Leu His Asn Ile
65                  70                  75                  80

Asp Val Asp Ala Gln Gln Phe Ser Asp Val Tyr Leu Glu Asn Leu Gly
                85                  90                  95

Lys Glu Val His Leu Ile Glu Gly Ala Val Gln Leu Cys Glu Asn Leu
            100                 105                 110

Gln Asp Cys Lys Leu Gly Ile Ile Thr Asn Gly Tyr Thr Lys Val Gln
        115                 120                 125

Gln Ser Arg Ile Gly Asn Ser Pro Leu Cys Asn Phe Phe Asp His Ile

130                 135                 140

Ile Ile Ser Glu Glu Val Gly His Gln Lys Pro Ala Arg Glu Ile Phe
145                 150                 155                 160

Asp Tyr Ala Phe Glu Lys Phe Gly Ile Thr Asp Lys Ser Ser Val Leu
                165                 170                 175

Met Val Gly Asp Ser Leu Thr Ser Asp Met Lys Gly Gly Glu Asp Tyr
            180                 185                 190

Gly Ile Asp Thr Cys Trp Tyr Asn Pro Ser Leu Lys Glu Asn Gly Thr
        195                 200                 205

Asp Val Asn Pro Thr Tyr Glu Val Glu Ser Leu Leu Gln Ile Leu Glu
    210                 215                 220

Ile Val Glu Val Ala Glu Glu Lys Val Ala Ser Phe
225                 230                 235

<210>    4
<211>    711
<212>    DNA
<213>    Bacillus cereus

<400>    4
atgaaataca aagttatatt attcgacgta gatgatacat tattagattt ccctgaaacg        60

gaaagacacg cattacataa tgcgtttgta cagtttgata tgcctacagg gtataatgat       120

tatcttgcaa gctataaaga gattagtaat ggattatgga gagatttaga aaataaaatg       180

attacgctaa gtgaattagc agtagatcga tttagacaat tatttgcact tcataatata       240

gacgtagatg cacagcaatt tagtgatgta taccttgaaa atttagggaa ggaagtacat       300

cttatagaag gcgcagtaca attatgtgaa aatctacaag attgcaagtt aggtattatt       360

acgaatggat atacgaaggt gcaacaatca agaatcggaa attcaccttt atgtaatttc       420

tttgatcaca ttattatttc tgaagaagtt ggtcatcaaa aaccagcacg tgagattttt       480

gattatgcgt ttgagaagtt tgggattact gataaatcaa gcgtactaat ggttggagat       540

tcgttaactt ctgatatgaa aggcggagaa gattacggca ttgatacgtg ttggtataat       600

ccgagtttga aagaaaacgg gacagatgtt aacccgactt atgaagtgga gagtctgctc       660

caaattttag aaattgtaga agtggcggaa gaaaaggtag cttcatttta a                711

<210>    5
<211>    231
<212>    PRT
<213>    Bacillus cereus

<400>    5
Met Lys Lys Tyr Lys Thr Leu Leu Phe Asp Val Asp Asp Thr Leu Leu
  1               5                  10                  15

Asp Phe Gln Lys Ala Glu Lys Val Ala Leu Arg Val Leu Phe Glu Glu
             20                  25                  30

Lys Gly Ile Pro Leu Thr Asp Glu Ile Glu Ala Arg Tyr Lys Lys Ile
        35                  40                  45

Asn Lys Gly Leu Trp Asp Ala Phe Glu Lys Gly Glu Leu Ser Arg Asn
    50                  55                  60

Glu Val Val Asn Thr Arg Phe Ser Leu Leu Phe Lys Glu Tyr Gly Glu
65                  70                  75                  80

Glu Val Asn Gly Ile Leu Phe Glu Asn Asn Tyr Arg Asn Tyr Leu Glu
                85                  90                  95

Glu Gly Asn Gln Leu Met Gln Gly Ala Phe Glu Phe Ile Asn Gln Ile
            100                 105                 110

Gln Gly Glu Tyr Glu Leu Tyr Ile Val Thr Asn Gly Val Ser Lys Thr
        115                 120                 125

Gln Asp Lys Arg Leu Arg Asn Ala Gly Leu His Ser Leu Phe Lys Asp
    130                 135                 140

Val Phe Val Ser Glu Asp Thr Gly Phe Gln Lys Pro Met Lys Glu Tyr
145                 150                 155                 160

Phe Asp Tyr Val Phe Glu Arg Ile Pro Asn Phe Ala Pro Glu Glu Gly
                165                 170                 175

Leu Ile Ile Gly Asp Ser Leu Ser Ala Asp Ile Lys Gly Gly Tyr Val
            180                 185                 190

Ala Gly Ile Asp Thr Cys Trp Phe Asn Pro Glu Arg Lys Leu Asn Asp
        195                 200                 205

Ser Gly Ile Ile Pro Thr Tyr Glu Val His Asn Phe Glu Glu Leu Glu
    210                 215                 220

Ala Leu Leu Lys Gln His Val
225                 230


<210>    6
<211>    696
<212>    DNA
<213>    Bacillus cereus

<400>    6
atgaaaaaat ataaaacatt gctatttgat gtagatgata cattattaga tttccaaaag      60

gctgaaaaag tggctttacg ggtgcttttt gaagagaagg gaatcccttt aacagacgag     120

atagaggctc gttataaaaa gataaataaa ggtctttggg atgcttttga aaaaggtgaa     180

ctatcacgca atgaagttgt aaatacacga ttctctctgt tgtttaaaga gtatggagaa     240

gaagtaaatg gaatattatt cgaaaataat tatcgtaact acttagaaga aggaaatcaa     300

ctcatgcaag gtgcatttga atttataaat caaattcaag gggagtatga attgtatata     360

gtgacaaatg gtgtttctaa gacgcaagat aaacgtttgc gcaatgcagg gttacattca     420

ttatttaaag atgtctttgt ttcggaagat acaggttttc aaaaaccgat gaaagagtat     480

tttgattatg tttttgaacg gattcctaac tttgcacctg aagaagggct gattattggg 540

gattcattaa gcgctgatat taaaggtgga tatgtagcgg gaattgatac ttgttggttt 600

aatccagaaa ggaaattaaa tgatagtggg attataccga catatgaagt gcataatttt 660

gaggagttag aagcgttatt gaagcagcat gtgtaa 696

<210> 7
<211> 247
<212> PRT
<213> Bacillus megaterium

<400> 7
Met Gln Lys Gln Thr Leu Leu Phe Asn Leu Asp Asp Thr Leu Val His
1               5                   10                  15

Cys Asn Lys Tyr Phe Arg Asp Thr Ile Asn Ala Phe Val Ala Gln Leu
            20                  25                  30

Gln Glu Trp Phe Glu Asn Leu Thr Lys Glu Glu Ile Lys Gln Lys Gln
        35                  40                  45

Leu Glu Ile Asp Leu Lys Ser Ile Glu Lys His Gly Leu His Ser Ser
    50                  55                  60

Arg Phe Pro Glu Ser Leu Val Ala Thr Tyr Leu Phe Phe Ser Glu Gln
65                  70                  75                  80

Asn His Gln Asp Ile His Glu Asp Arg Val Gln Arg Val Lys Lys Ile
                85                  90                  95

Gly Gln Ser Val Phe Glu Val Pro Ile Glu Pro Leu Pro Asn Met Tyr
            100                 105                 110

Glu Val Leu Asp Lys Leu Lys Glu Gln Gly His Asp Leu Tyr Leu His
        115                 120                 125

Thr Gly Gly Asp Glu Glu Asn Gln Gly Arg Lys Ile Val Gln Leu Glu
    130                 135                 140

Leu Ala Lys Tyr Phe Glu Lys Arg Val Phe Ile Ser Glu Gln Lys Asp
145                 150                 155                 160

Thr Ala Ala Leu Lys Glu Ile Leu Asn Gln Ile Lys Tyr Asp Pro Lys
                165                 170                 175

Lys Thr Trp Met Ile Gly Asn Ser Leu Lys Thr Asp Ile Arg Pro Ala
            180                 185                 190

Val Glu Ala Gly Ile His Ala Ile His Val Pro Ser Glu Leu Glu Trp
        195                 200                 205

Ser Tyr Asn Gln Val Lys Ile Asp Val Ala Pro Lys Gly Lys Leu Ile
    210                 215                 220

Thr Val Asn Ser Leu Leu Glu Val Pro Glu Ile Ile Gln Lys His Ala
225                 230                 235                 240

Gln Glu Thr Val Val Ser Thr
                245

<210>    8
<211>    744
<212>    DNA
<213>    Bacillus megaterium

<400>    8
atgcagaagc aaaccctact ttttaattta gacgatacgc ttgttcactg taataaatat      60

tttagagaca ccataaatgc ttttgtggct caattgcagg aatggtttga gaatctgact     120

aaggaagaaa taaaacaaaa gcagttagag atagatttaa aaagtatcga aaagcatggc     180

cttcactctt cccgatttcc agaatcatta gtagctactt atttattttt tagtgaacaa     240

aatcatcaag atatccatga agatagagtc caaagagtca aaaaaattgg tcaaagtgta     300

tttgaagtac cgatagagcc tttgccaaat atgtatgaag tattagataa gcttaaagaa     360

caaggacatg atctctactt acacacaggt ggagacgagg aaaatcaagg tagaaaaatt     420

gttcagctag agctagctaa atactttgaa aaaagggtat ttatttctga acaaaaagat     480

acagctgcat taaaagaaat actaaaccaa attaagtacg atccaaaaaa gacttggatg     540

attggcaatt cattaaaaac cgatataaga ccggcagtag aagctggaat acatgcgatt     600

cacgtacctt ctgaactgga gtggagctac aaccaagtta aaattgatgt tgcgccaaaa     660

ggtaaattga taaccgttaa ttcgttgctg gaagttccag aaatcattca aaagcatgct     720

caagaaacgg ttgtgtccac ctaa                                            744


<210>    9
<211>    219
<212>    PRT
<213>    Bacillus megaterium

<400>    9
Met Lys Phe Lys Thr Ile Leu Phe Asp Ala Tyr Gly Thr Leu Phe Asp
1               5                   10                  15

Val His Thr Val Ile Glu Thr Cys Asp Lys Leu Tyr Pro Glu Arg Gly
            20                  25                  30

Glu Ser Ile Ser His Leu Trp Arg Leu Lys Gln Ile Glu Tyr Ala Met
        35                  40                  45

Gln Tyr Gln Leu Met Gly Arg Tyr Val Asp Phe Tyr Thr Leu Thr Asn
    50                  55                  60

Gln Ser Leu Lys Tyr Ala Ala Glu Ala Asn Asp Ile Asp Leu Thr Glu
65                  70                  75                  80

His Glu Glu Lys Met Leu Met Ser Ala Tyr Met Lys Leu Asn Val Tyr
                85                  90                  95

Asp Glu Val Lys Glu Val Leu Ala Tyr Leu Lys Glu Lys Gly Tyr Arg
            100                 105                 110

Leu Ala Ile Phe Thr Asn Gly Pro Lys His Met Ile Asp Pro Leu Val
        115                 120                 125

Ser Tyr His Asn Met Asn His Leu Phe Glu Asp Val Ile Ser Val Asp
    130                 135                 140

Glu Ile Lys Gln Tyr Lys Pro Thr Met Ala Ser Tyr His Tyr Ala Lys
145                 150                 155                 160

Asn Lys Met Asn Ala Lys Arg Glu Glu Val Leu Phe Leu Ser Ser Asn
                165                 170                 175

Thr Trp Asp Ile Ala Gly Ala Lys Asn Tyr Gly Phe Ala Thr Ala Trp
            180                 185                 190

Val Asn Arg Lys Gly Asn Val Ala Glu His Lys Glu Leu Gln Pro Asn
        195                 200                 205

Val Ile Ile Lys Ser Leu Asn Gln Leu Ile Lys
    210                 215


<210>    10
<211>    660
<212>    DNA
<213>    Bacillus megaterium

<400>    10
atgaaattta aaacgatttt atttgatgca tatggaacgc tgtttgatgt acataccgtt          60

attgaaacgt gtgataagct gtatccggag cggggagaaa gtattagcca tttatggcgt         120

ttgaagcaaa ttgaatatgc aatgcaatat cagctgatgg gacgatacgt tgacttttat         180

acattaacaa atcaatccct aaaatatgca gcagaagcaa atgatattga tttaacggaa         240

catgaagaaa aaatgctgat gagtgcttac atgaagctga atgtctacga tgaggtcaaa         300

gaggtgctcg cctatttaaa agaaaaagga tatcgtctgg ctatttttac gaatggtcct         360

aagcacatga ttgatccgct tgtttcttat cacaacatga atcatttatt tgaagacgtt         420

atttccgtag acgaaatcaa acagtacaag cctacgatgg caagctatca ctatgcaaaa         480

aataaaatga acgcaaaaag agaagaagta ttatttttgt catcgaatac atgggatatt         540

gccggtgcta aaaactacgg gttcgcaacg gcttgggtca accgaaaagg aaatgtagct         600

gaacataaag aattgcagcc aaacgtaatc attaaaagct taaatcagct tattaaataa         660

                                                                          660


<210>    11
<211>    238
<212>    PRT
<213>    Bacillus megaterium

<400>    11
Met Lys Thr Tyr Arg Thr Leu Leu Phe Asp Ile Asp Asn Thr Leu Leu
  1               5                  10                  15

Asp Phe Asn Ala Ala Glu Glu Gln Ala Leu Gln Leu Leu Phe Ala Asn
             20                  25                  30

His Asp Ile Pro Leu Thr Glu Glu Ser Lys Lys Arg Tyr Ser Leu Ile
        35                  40                  45

Asn Gln Gly Leu Trp Thr Ala Phe Glu Glu Asn Lys Ile Ser Arg Glu
    50                  55                  60

Gln Val Val Asn Thr Arg Phe Ser Thr Leu Cys Lys Glu Tyr Gly Ile
65                  70                  75                  80

Glu Lys Asp Gly Lys Leu Leu Glu Ala Glu Tyr Arg Thr Tyr Leu Asn
                85                  90                  95

Asn Gly His Gln Leu Ile Asp Gly Ala Phe Glu Val Ile Lys Asn Leu
            100                 105                 110

Ser Cys His Tyr Glu Leu Tyr Val Val Thr Asn Gly Val Ser Ala Thr
        115                 120                 125

Gln Tyr Lys Arg Leu Gln Asp Ser Gly Leu Tyr Pro Tyr Phe Lys Glu
    130                 135                 140

Val Phe Val Ser Glu Asp Thr Gly Phe Gln Lys Pro Met Lys Glu Tyr
145                 150                 155                 160

Phe Asp Tyr Val Phe Ser Arg Ile Thr Glu Leu Ser Val His Glu Thr
                165                 170                 175

Leu Ile Ile Gly Asp Ser Leu Ser Ala Asp Ile Gln Gly Gly Gln Leu
            180                 185                 190

Ala Gly Ile Asp Thr Cys Trp Phe Asn Pro Gln Lys Lys Lys Asn Asp
        195                 200                 205

Thr Asn Trp Asn Ser Thr His Glu Ile Gly Arg Leu Gln Glu Leu Tyr
    210                 215                 220

Ile Leu Leu Asn Val Asn Asn Glu Val Ala Ser Val Gln Val
225                 230                 235


<210>    12
<211>    717
<212>    DNA
<213>    Bacillus megaterium

<400>    12
atgaaaacat accgaacgct tctatttgac attgataata cgcttttaga ttttaacgca        60

gccgaagagc aggctttaca gcttcttttt gctaaccacg atattccgtt gactgaggaa       120

agcaaaaaac gttatagttt gataaaccag gggttatgga cagcttttga agaaaataaa       180

ataagccgtg agcaagttgt aaatactcgc ttttctactc tatgtaaaga atatggaata       240

gaaaaagacg gaaagctact tgaagcagag taccgaacgt atttgaacaa tggccatcag       300

ctaattgacg gagcatttga ggtaattaaa aacctgagct gtcactatga gttatatgtt       360

gttacaaatg gcgtatcggc tacgcaatat aaacgccttc aggactcagg tctatatcct       420

tattttaaag aagtatttgt ttcagaagat acgggctttc aaaagcctat gaaggaatat       480

tttgattatg ttttttcacg tataacagag ttgtccgtac atgaaacatt aattattgga       540

gattctttga gtgcagacat tcaaggcggg cagctagccg gtattgatac gtgttggttt 600

aatccgcaga aaaaaaagaa tgatacaaat tggaattcta cccatgaaat aggaaggctt 660

caagagcttt atattctgct gaacgtaaat aatgaagtag cttcggtaca agtgtaa 717

<210> 13
<211> 38
<212> DNA
<213> Artificial Sequence

<220>
<223> primer

<400> 13
aagaaggaga tataccatga tgggttataa agcgatgc 38

<210> 14
<211> 41
<212> DNA
<213> Artificial Sequence

<220>
<223> primer

<400> 14
gcattatgcg gccgcaagct ttacttgtta taaaaatatt g 41

<210> 15
<211> 40
<212> DNA
<213> Artificial Sequence

<220>
<223> primer

<400> 15
aagaaggaga tataccatga aatacaaagt tatattattc 40

<210> 16
<211> 41
<212> DNA
<213> Artificial Sequence

<220>
<223> primer

<400> 16
gcattatgcg gccgcaagct ttaaaatgaa gctacctttt c 41

<210> 17
<211> 38
<212> DNA

<213>    Artificial Sequence

<220>
<223>    primer


<400>    17
aagaaggaga tataccatga aaaaatataa aacattgc                                38


<210>    18
<211>    40
<212>    DNA
<213>    Artificial Sequence

<220>
<223>    primer


<400>    18
gcattatgcg gccgcaagct ttacacatgc tgcttcaata                              40


<210>    19
<211>    37
<212>    DNA
<213>    Artificial Sequence

<220>
<223>    primer


<400>    19
aagaaggaga tataccatgc agaagcaaac cctactt                                 37


<210>    20
<211>    41
<212>    DNA
<213>    Artificial Sequence

<220>
<223>    primer


<400>    20
gtggtggtgg tggtgctcga ttaggtggac acaaccgttt c                            41


<210>    21
<211>    37
<212>    DNA
<213>    Artificial Sequence

<220>
<223>    primer


<400>    21
aagaaggaga tataccatga aatttaaaac gatttta                                 37


<210>    22

<211>    40
<212>    DNA
<213>    Artificial Sequence

<220>
<223>    primer


<400>    22
gtggtggtgg tggtgctcga ttatttaata agctgattta                              40


<210>    23
<211>    36
<212>    DNA
<213>    Artificial Sequence

<220>
<223>    primer


<400>    23
aagaaggaga tataccatga aaacataccg aacgct                                  36


<210>    24
<211>    41
<212>    DNA
<213>    Artificial Sequence

<220>
<223>    primer


<400>    24
gtggtggtgg tggtgctcga ttacacttgt accgaagcta c                            41